Europäisches Patentamt

European Patent Office

Office européen des brevets

(11) Veröffentlichungsnummer: **0 307 500**
**A1**

## (12) EUROPÄISCHE PATENTANMELDUNG

(21) Anmeldenummer: 87113610.7

(22) Anmeldetag: 17.09.87

(51) Int. Cl.⁴: **C12M 1/00**

(43) Veröffentlichungstag der Anmeldung:
22.03.89 Patentblatt 89/12

(84) Benannte Vertragsstaaten:
AT BE CH DE ES FR GB GR IT LI LU NL SE

(71) Anmelder: INREVERS VERWALTUNGS
G.M.B.H.
Gräsiger Weg 7a
D-6238 Hofheim-Wallau(DE)

(72) Erfinder: Lotz, Horst K.
Fasanenweg 7
D-6240 Königstein-Johanniswald(DE)

(54) **Transportabler Vielfalt-Biogas-Erzeuger.**

(57) Zum umweltschützenden und preiswerten Erzeugen von Biogas aus in Art und Anfallort unterschiedlich verfügbarer Biomasse, insbesondere auch für kleine Mengen und für Bereiche mit wirtschaftlich nur schwer beschaffbarer Energie, wird ein neuartiger, vorzugsweise transportabler, vielfältig einsetzbarer Biogas-Erzeuger vorgestellt, der in entsprechenden Stückzahlen und Ausführungen fabrikmäßig hergestellt wird.

Bild 1

EP 0 307 500 A1

### Transportabler Vielfalt-Biogas-Erzeuger

Seit geraumer Zeit ist die Erzeugung von zur Heizung oder als Brennstoff für Gasmotoren geeigneten Gasen durch Vergären von organischen Stoffen wie Pflanzen, Abfällen aus Viehwirtschaft und Nahrungsmittelerzeugung und ähnlichen bekannt. Auch erlaubt erst eine biologische Behandlung von Abfallstoffen und Rückständen in Industrie und Land- bzw. Viehwirtschaft eine umweltfreundliche Entsorgung bzw. Weiterverwendung derselben. Es gibt eine Reihe von einfachen bis zu komplizierten Verfahren und zugehörigen Einrichtungen, um mit den jeweiligen Biomassen oder -mengen günstige Ergebnisse und eine wirtschaftlichste Nutzung zu erzielen. Auch versucht man durch Anbau und Verwendung besonders energiereicher Pflanzen eine Alternative zu den bekannten Energieträgern zu schaffen. Alle soweit bekannten Einrichtungen zur Biogas-Erzeugung sind ortsfest installierte, relativ große Einheiten, die nahe des Anfallortes der Biomasse z.B. in einem landwirtschaftlichen Betrieb fest installiert und verrohrt sind.

Nachstehende Prinzipdarstellung eines Fermenters (Illustration 1) zeigt eine ursprüngliche Ausführung, die alle wesentlichen Merkmale eines nach dem Batch-Verfahren arbeitenden Fermenters hat. Das Flußdiagramm (Illustration 2) erläutert, daß vor und nach dem Biogas-Erzeuger (-fermenter) weitere wesentliche Anlageneinrichtungen für einen vorteilhaften Betrieb zu bedenken sind.

Illustration 1

Illustration 2

Kennzeichnend für diese sehr unterschiedlichen Anlagen - die diskontinuierlich, kontinuierlich, mit oder ohne Speichersystem beschichtet, im Durchflußverfahren mit senkrechter oder waagrechter Strömung in einem oder 2 Fermentergehäusen betrieben, mit Heizsystemen und mit Mischsystemen verschiedener Ausführung sowie mit Rührwerken und Einrichtungen zum Speichern und Reinigen von Gas ausgestattet sind - ist ihre Ausführung für große spezifische Anwendungsfälle, d.h. für in großer Menge an gleichem Ort anfallende Menge in etwa gleicher Biomasse.

Für die neuesten in Betrieb befindlichen Anlagen gilt die Annahme der Unwirtschaftlichkeit. Und das aus zwei Hauptgründen:

a) Die schlechte Betriebsweise mit Versorgung mit Biomasse und mit weitgehender regelmäßiger Nutzung des Biogases.

b) Die mangelnde Wirtschaftlichkeit wegen der hohen Investitions- bzw. Baukosten.

Meist wurden solche Anlagen wegen der örtlichen Verfügbarkeit größerer Mengen organischer Stoffe oder Reststoffe, wegen behördlichen Umweltschutzauflagen, zu Forschungszwecken oder einfach aus persönlicher Begeisterung für diese Art der Energieerzeugung bzw. des sinnvollen Umweltschutzes verwendet.

Unter Berücksichtigung der Wirtschaftlichkeit, oder biologischen, chemischen, physikalischen und technischen Voraussetzungen haben sich eine Reihe bedarfsorientierter Verfahren und Systeme herausgebildet, die aus nachstehender Aufstellung hervorgehen (Illustration 3):

Merkmale bestehender Gärverfahren

| System | Verfahrensmerkmale | Schema |
|---|---|---|
| 1.Batch-System | Verfahren, das durch eine diskontinuierliche, einmalige Beschickung ohne gleichzeitige Entleerung des Fermenterinhaltes charakterisiert ist. Ein Rührwerk kann vorhanden sein. | |
| 2.Speichersystem | Verfahren, das durch diskontinuierliche, mehrmalige Beschickung (a,b,c,d) ohne gleichzeitige Entleerung des Fermenterinhaltes charakterisiert ist. | |
| 3.Vollständig durchmischtes, kontinuierliches System | Einstufiges Durchflußverfahren mit Rührsystem zur Homogenisierung des Faulrauminhaltes. Die Beschickung erfolgt kontinuierlich oder semikontinuierlich. | |

Merkmale bestehender Gärverfahren (Fortsetzung)

| System | Verfahrensmerkmale | Schema |
|---|---|---|
| 4. Teilweise durchmischtes, kontinuierliches System | Es unterscheidet sich vom vollständig durchmischten Verfahren durch die unvollständige Homogenisierung des Behälterinhaltes. | |
| 5. Kontinuierliches Gärkanalsystem | Einstufiges Durchflußverfahren mit horizontaler Strömung. Es kann ein Rührwerk vorhanden sein. | |
| 6. Anaerobes Kontaktverfahren | Es ist identisch mit dem vollständig durchmischten, kontinuierlichen Verfahren, jedoch wird die aktive Biomasse aus der Sedimentationsstufe zurückgeführt. | |
| 7. IRIS-System | Dieses Verfahren wurde vom Institut de Recherches de 1'Industrie Sucrière entwickelt. Das Verfahren gleicht dem Anaeroben Kontaktverfahren, jedoch findet die Sedimentation im Methanfermenter statt. | |
| 8. Aufstromsystem mit Schlammbett ohne Trägermaterial | Aufstrom-Biogasvrfahren mit integriertem Schlamm- und Gasabscheider. Der sedimentierte Schlamm verbleibt als Schlammbett im unteren Teil des Fermenters. | |
| 9. Anaerob-Filter | Auf- oder Abstromverfahren, bei dem die aktive Biomasse zurückgehalten und auf einem festen Trägermaterial (Fixed Film) angesiedelt wird. | |
| 10. Wirbelbett-System (Fluidized Bed) | Aufstromverfahren mit Zurückhaltung und Ansiedlung der aktiven Biomasse auf Kleinstfüllkörpern, die durch Kreislaufführung der Flüssigkeit in der Schwebe gehalten werden. | |
| 11. 2-stufiges System | Gärverfahren in zwei getrennten hintereinandergeschalteten Fermentern. Die Hydrolyse (Acidogenese) findet im ersten Fermenter, die Methanogenese im zweiten Fermenter statt. Jeder für sich kann nach oben beschriebenen Verfahren gestaltet sein. | |

Die sinnvolle Nutzung einer Biogas-Anlage verlangt einen ausreichend großen Anfall von Biomasse oder eine mehr oder weniger angemessene Abnahme und Nutzung von Gas und der als Dünger verwertbaren, überwiegend flüssigen und schlammartigen Rückstände.

Bei einem vielleicht auch ungleichmäßigen, geringen Anfall von verwertbarer Biomasse unterschiedlicher Art an verschiedenen Orten und zu verschiedenen Zeiten, ebenso wie eine nicht gleichmäßige und nur an verschiedenen Orten erfolgende Abgabe von Gas und Rückständen besteht ein Bedarf an vielen kleinen Erzeugeranlagen, was vom Aufwand und der Wirkung her als sehr unwirtschaftlich zu betrachten ist, es sei denn, sie seien preiswert für verschiedenste Anwendung und leicht veränderbaren Nutzungsort gebaut.

Die nachstehend beschriebene Erfindung beansprucht die Nutzung eines dezentralisierten oder gar ortsveränderlichen Anfalls von geringen Biomassemengen wirtschaftlich zu ermöglichen. Ein mit Füll- und Entleermöglichkeit ausgestatteter, in Größe und Form transportabler Behälter, der mit Isolierung, Heizung, Durchmischungsmöglichkeit, Gassammelbereich, Gasreinigung und Meß-bzw. Zugabemöglichkeiten, ja sogar schon mit Gasverwertung ausgestattet ist, wird auf ein schleppbares oder selbstgetriebenes Fahrgestell gesetzt, um zum Aufnehmen von Biomassen oder zum Abgeben von Gas und Rückständen von Ort zu Ort bewegt zu werden. Zerkleinerungsmaschinen, Sonnenkollektoren oder andere Beheizungsmöglichkeiten, Gasabfüllpumpen und Gasbehälter sind zugeordnet und erlauben jeweils eine günstige Verfahrensgestaltung und komprimierte Gasabgabe je nach den gegebenen Umständen. Es können gemäß der Erfindung einfache und primitive Kleinanlagen für einfachste Bedürfnisse mit Gasflaschenfüllung bis hin zu kompletten Generatoranlagen mit Motoren und Stromerzeugern preiswert in entsprechenden Stückzahlen erstellt und vielseitig genutzt werden.

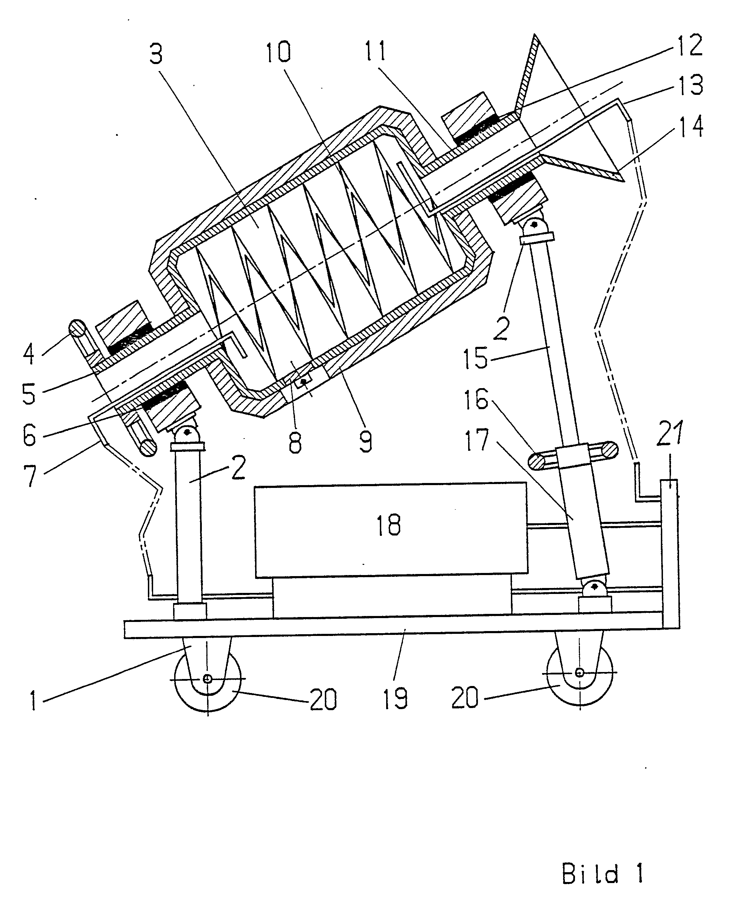

Eine erfindungsgemäße Ausführung ist in Bild 1 dargestellt. Auf einem Fahrwerk 1, ausgestattet mit einem Hubwerk 2, auch als in der Höhe unveränderliche Drehlagerung verstellbar, befindet sich eine zur Fermentierung der Gärung, genauer zur Methangenese vorgesehener Behälter 3.

Im Einzelnen ist dieser mit einem als Handrad ausgebildeten Drehantrieb (4) am auch als Achsstummel für das Drehen des Behälters und zum Entleeren dienenden unteren Behälterhals (5) ausgerüstet. Dieser Behälterhals (5) ruht in einem Lager (6) unten, um ein Drehen des Behälters (3) zu ermöglichen. Durch den Behälterhals (5) ragen eine Sonde oder Zuführung (7) oder auch mehrere zum Messen, Entnehmen oder Zuführen in das Behälterinnere. Für eine mögliche diskontinuierliche Entleerung ist im unteren Bereich des Behälters (3) eine Entleeröffnung (8) vorgesehen. Um die Verfahrenstemperatur im gewünschten Bereich von etwa 30 - 35 ° C oder von über 50 °C wirtschaftlich zu halten, ist der Behälter (3) mit einem Isolationsmantel (9) aus wärmedämmendem Material umgeben. An der inneren Wand des Behälters (3) ist eine Wendel (10) zum Durchmischen der Biomasse und zum Zerbrechen einer Schwimmdecke bzw. zum Auflösen einer Sedimentschicht bei Drehungen in beiden Richtungen oder zum Transport bei flacher Winkelstellung eingeschweißt.

Der obere Behälterhals (11) ruht wieder im zum Drehen vorgesehenen Lager (12), das mit der Hubspindel (15), der Hubspindelmutter (16) mit Handrad, beides im und am Hubspindelgehäuse (17), in der Höhe verstellt werden kann.

Durch den oberen Behälterhals (11) führen Sonden, Zuführ- und Gasentnahmeleitungen (13) in den Behälter (3). Zudem ist dem Behälterhals (11) ein Einfüllstutzen (14) aufgesetzt.

Am Behälter (3) oder auf dem Fahrwerksrahmen (19) mit den Fahrwerksrädern (20) ist ein Sonnenkollektor (18) zur Beschaffung warmen Wassers für die Erzeugung einer geeigneten Verfahrenstemperatur im Behälter (3) montiert. Auch ist das Fahrwerk 1 mit einer Meß- und Einstellstation (21) verbunden.

Legende zu Bild 1:

1 Fahrwerk
2 Hubwerk mit Drehlager
3 Behälter
4 Drehantrieb (Handrad)
5 Behälterhals, unten
6 Lager, unten
7 Sonde oder Zuführung, unten
8 Entleeröffnung
9 Isolationsmantel
10 Misch- und Transportwendel
11 Behälterhals, oben

12 Lager, oben

13 Sonde, Zuführung oder Gasentnahme, oben

14 Einfüllstutzen

15 Hubspindel

16 Hubspindelmutter mit Handrad

17 Hubspindelgehäuse

18 Sonnenkollektor

19 Fahrwerkrahmen

20 Fahrwerkräder

21 Meß- und Einstellstation

## Ansprüche

1) Vielfalt-Biogas-Erzeuger zum variablen Einsatz nach einem der oder einer Kombination von bekannten Verfahren, mit verschiedenartiger Biomasse, mit verschiedenartigen Verfahren, unter verschiedenen Umweltbedingungen, für verschiedene Mengen und an verschiedenen Orten, dadurch gekennzeichnet, daß ein fest oder abnehmbar mit einem gezogenen oder selbsttreibenden Fahrwerk oder Schwimmkörper verbundener, länglicher, schräg angeordneter oder zwischen waagrecht und senkrecht einstellbarer Erzeugerbehälter je nach Bedarf in abgestuften Größen, leicht mit einfachen bzw. behältereigenen Hilfen füllbar, entleerbar oder den Inhalt umwälzbar, heizbar, befeuchtbar mit Zusätzen beschichtbar und wärmeisoliert ausgeführt ist, und daß verfahrenbeginnende bzw. -unterstützende Mittel an geeigneten Stellen, zu geeigneten Zeiten in geeigneten Formen und dosierten Mengen zugeführt und die anfallenden Gase, Flüssigkeiten und Feststoffe in dafür besonders vorgesehenen Raumbereichen gesammelt von dort über Filterung, Reinigung mit oder ohne physikalische oder chemische Veränderung abgenommen oder abgesaugt, komprimiert und in separate Einzel-oder Sammelbehälter bzw. gegebenenfalls über lösbare Anschlüsse in Leitungen verbracht werden. Auf dem Fahrwerk oder Schwimmkörper bzw. auf einem zugeordneten mitfahrenden bzw. angehängten Fahrwerk oder Schwimmkörper sind gasverbrauchende Aggregate wie Brenner oder Motoren mit energieaufnehmenden und -umwandelnden Geräten wie z.B. Elektrizitätsgeneratoren mit Batterien oder Wärmespeichern angebracht. An den entsprechenden Stellen des Erzeugers oder der Zusatzgeräte oder der Gasverbraucher sind Verfahrens- und ergebniskontrollierende Meßinstrumente für Druck, Temperatur, Menge, Feuchtigkeit, chemische Analyse, Stromstärke, Spannung usw. angeordnet, um den Verfahrensablauf manuell bzw. automatisch zu steuern, auch damit die Verfahrensrückstände umweltfreundlich und nutzbringend entsorgt werden können.

2) Vielfalt-Biogas-Erzeuger zum variablen Einsatz nach einem der oder einer Kombination von bekannten Verfahren, mit verschiedenartiger Biomasse, mit verschiedenartigen Verfahren, unter verschiedenen Umweltbedingungen, für verschiedene Mengen und an verschiedenen Orten nach Anspruch 1, dadurch gekennzeichnet, daß Form, Lage, Isolation und Ausstattung des Erzeugerbehälters eine leichte und schnelle Beschickung oder Entsorgung der Reste und einen schnellsten Verfahrensablauf bei Teilfüllung, Ganzfüllung und kontinuierlichem Füll- oder Entsorgungsbetrieb erlauben, indem ein drehbarer, im Einzeln durch Hubspindel höhenverstellbaren Drehlagern abgestützter zylindrischer Behälter mit Einfüllstutzen oben und Entleeröffnung unten, mit Misch- und Transportwendel innen sowie einem Isolationsmantel außen, dazu auch mit durch die als Drehachsstummel ausgebildeten Behälterhälse oder direkt in die entsprechenden Behälterbereiche geführten Meßsonden und Zufuhr-bzw. Entnahmeleitungen ausgestattet ist. Auf dem Fahrwerk sind außer den verschiedenen Haltern und Lagern für Rohr- und Schlauchleitungen bzw. Hubwerk eine Meß- und Einstellstation und ein Sonnenkollektor für die Beheizung des Erzeugerbehälters angeordnet.

3) Vielfalt-Biogas-Erzeuger zum variablen Einsatz nach einem der oder einer Kombination von bekannten Verfahren, mit verschiedenartiger Biomasse, mit verschiedenartigen Verfahren, unter verschiedenen Umweltbedingungen, für verschiedene Mengen und an verschiedenen Orten nach den Ansprüchen 1 und 2, dadurch gekennzeichnet, daß je nach Verfahrensart, Biomasse, Fahrwerks-oder Schwimmkörpergröße bzw. -ausführung der Biogas-Erzeuger aus zwei oder mehr entsprechend ausgeführten und ausgestatteten Behältern besteht, die nebeneinander, übereinander oder hintereinander angeordnet und für einen parallelen oder abschnittsweisen bzw. schrittweisen Betrieb in verschiedenen Lagen zueinander und mit Vorrichtungen für den Biomassendurchfluß oder -transport ausgerüstet sind. Die Behälter sind einzeln oder gemeinsam oder wechselweise drehbar oder verstellbar ausgeführt.

4) Vielfalt-Biogas-Erzeuger zum variablen Einsatz nach einem der oder einer Kombination von bekannten Verfahren, mit verschiedenartiger Biomasse, mit verschiedenartigen Verfahren, unter verschiedenen Umweltbedingungen, für verschiedene Mengen und an verschiedenen Orten nach den Ansprüchen 1 bis 3, dadurch gekennzeichnet, daß der Erzeugerbehälter für die Entleeröffnung im oder am unteren oder oberen Behälterhals einen Deckel oder Verschluß hat, der eine kontinuierliche oder diskontinuierliche manuelle oder automatische Beschickung mit bzw. Entleerung von flüssigen, schlammigen oder festen Biomassen, aktiven Biomassen, Zusätzen und Rückständen ermöglicht. Der Behälter ist in Schräglage fest, aber um seine Längsachse drehbar angeordnet und im Innern mit Zwischenfiltern, Trennwänden, Katalysatoren oder Trägermitteln für verfahrensbedingte oder -fördernde chemische oder biologische Stoffe versehen.

5) Vielfalt-Biogas-Erzeuger zum variablen Einsatz, mit verschiedenartigen Verfahren, mit verschiedenartiger Biomasse, unter verschiedenen Umweltbedingungen, für verschiedene Mengen und an verschiedenen Orten nach den Ansprüchen 1 bis 4, dadurch gekennzeichnet, daß für alle handbetriebenen Bewegungen und Verstellungen im Einzelnen oder als gesteuerter bzw. geregelter Funktionsablauf bekannte pneumatische, hydraulische oder elektromotorische Antriebe mit an sich bekannten Steuer- und Regelkreisen eingesetzt werden.

6) Vielfalt-Biogas-Erzeuger zum variablen Einsatz, mit verschiedenartigen Verfahren, mit verschiedenartiger Biomasse, unter verschiedenen Umweltbedingungen, für verschiedene Mengen und an verschiedenen Orten nach den Ansprüchen 1 bis 5, dadurch gekennzeichnet, daß zur Verfahrensunterstützung eine der Zusammensetzung und Menge der Biomasse im Erzeuger geeignete Temperatur zur Erwärmung mittels Wärmetauscher im Behälter angeordnet sind, durch die ein geeigneter Wärmeträger z.B. Wasser zuvor im Sonnenkollektor oder durch Biogas oder fremden Heizstoff erwärmt in geeigneter Menge mit entsprechender Temperatur fließt. Als Wärmetauscher kann neben beliebigen Heizkörpern wie z.B. Rohrschlangen auch der Behälter ganz oder teilweise als Doppelmantel ausgeführt werden. Besonders ist die Misch- und Transportwendel hohl, z.B. als Rohrschlange für den Heizmitteldurchlauf ausgebildet. Auch ist der Behälter mit einer ganz durchgehenden Hohlachse versehen die befüllbar, entleerbar, in Teilabschnitten verschließbar und in verschiedenen Behälterbereichen mit verschließbaren Zufuröffnungen zum Behälterinnern hin ausgeführt ist und auf dem Außenmantel die Misch- Transport- und Heizwendel führt.

7) Vielfalt-Biogas-Erzeuger zum variablen Einsatz, mit verschiedenartigen Verfahren, mit verschiedenartiger Biomasse, unter verschiedenen Umweltbedingungen, für verschiedene Mengen und an verschiedenen Orten nach den Ansprüchen 1 bis 6, dadurch gekennzeichnet, daß der Erzeugerbehälter auf einem Fahrwerk oder einem Schwimmkörper normalerweise einem Schiff oder Boot mit besonderen Entleerungsleitungen oder Zwischenbehältern zur ganzen oder teilweisen kontinuierlichen Entsorgung oder zur diskontinuierlichen Entsorgung je nach Zeit, Ort oder Wiederverwendungsort ausgerüstet ist.

8) Vielfalt-Biogas-Erzeuger zum variablen Einsatz, mit verschiedenartigen Verfahren, mit verschiedenartiger Biomasse, unter verschiedenen Umweltbedingungen, für verschiedene Mengen und an verschiedenen Orten nach den Ansprüchen 1 bis 7, dadurch gekennzeichnet, daß für das Vorbereiten der Biomasse für den Gärvorgang geeignete Zerkleinerungs- und Mischgeräte mit Befeuchtungs- und Zusatzdosiereinrichtungen mit oder ohne direkte Einleitung in dem Erzeugerbehälter vorhanden sind.

Bild 1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X,Y | FR-A-2 461 747 (A. MAUMONT) <br> * Anspruch 1; Figur 1 * <br> --- | 1,2,5-7 | C 12 M 1/00 |
| Y | FR-A-2 485 035 (A. MAUMONT) <br> * Ansprüche 1,2; Figur 1 * <br> --- | 6 | |
| X | FR-A-2 522 013 (L.P. STOTZ et al.) <br> * Seite 4, Zeilen 20-25; Ansprüche 1-9; Figur * <br> --- | 1,2,4-7 | |
| X | DE-A-2 535 756 (H. BESLER) <br> * Seite 15, Zeilen 12-20; Ansprüche 1-13; Figur * <br> --- | 1-3,5,8 | |
| A | DE-A-3 228 895 (SCHNEIDER) <br> * Seite 11, Zeilen 1-5; Ansprüche 1-12; Figuren 1-3 * <br> ----- | 1,2,4-6 | |
| | | | RECHERCHIERTE SACHGEBIETE (Int. Cl.4) |
| | | | C 12 M |

Der vorliegende Recherchenbericht wurde für alle Patentansprüche erstellt

| Recherchenort | Abschlußdatum der Recherche | Prüfer |
|---|---|---|
| DEN HAAG | 25-05-1988 | COUCKE A.O.M. |